# FASCICULE DE BREVET EUROPEEN

(11) **EP 0 453 378 B1**
(45) Date de publication et mention de la délivrance du brevet: **01.06.1994**
(21) Numéro de dépôt: 91420106.6
(22) Date de dépôt: 28.03.1991
(51) Int. Cl.: A61M 39/00

(54) **Structure de protection pour site de connexion à usage médical**
Schutzvorrichtung für eine Anschlussstelle für medizinische Zwecke
Protective structure for connection site for medical use

(30) Priorité: 20.04.1990 FR 9005351
(43) Date de publication de la demande: 23.10.1991
(73) Titulaire: CAIR (SOCIETE ANONYME DE DROIT FRANCAIS), F-69170 Tarare (FR)
(72) Inventeur: Lopez, Georges Antoine, F-69290 Craponne (Rhône) (FR)
(74) Mandataire: Perrier, Jean-Pierre

(56) Documents cités:
- EP-A- 0 073 432
- EP-A- 0 336 853
- FR-A- 2 617 717
- US-A- 4 896 465

## Description

De nombreux liquides médicaux sont administrés aux patients par voie parentérale, qu'il s'agisse de médicaments ou de sérums d'alimentation. Le patient est généralement équipé d'un cathéter engagé dans une veine, dans lequel débouche une tubulaire d'amenée des liquides. Afin d'adapter le traitement à l'état instantané du patient, il est nécessaire de pouvoir injecter simultanément ou en alternance plusieurs liquides. A cet effet, la ligne est équipée, à son extrémité opposée à celle comportant le cathéter, d'un site de connexion de plusieurs tubulures comportant un robinet à trois voies ou une rampe de robinets.

Afin d'éviter les risques de contamination du patient, il est nécessaire de maintenir ce site en conditions stériles.

A cette fin, il est connu, notamment dans EP-A-336 853, qui décrit une structure de protection pour site de connexion à usage medical possédant les caractéristiques correspondant aux préambule de la revendication 1, d'assurer cette protection au moyen d'un boîtier composé de deux demi-coquilles en matière synthétique articulées par leurs parois longitudinales postérieures, munies sur leurs parois longitudinales antérieures de moyens de fermeture élastique et munies de garnissages en mousse, destinés à être imbibée par un agent antiseptique et comportant des empreintes pour l'encastrement d'au moins un robinet et des tubulures du site. Les parois du boîtier comportent des ouvertures pour le passage des tubulures.

En général, le boîtier comporte, dans sa paroi opposée à celle munie des moyens de fermeture, une fente pour laisser le passage à une palette solidaire du site de connexion et servant à la fixation de celui-ci sur un support fixe disposé sur une potence. Actuellement, cette fixation est assurée par une pince qui, passant à travers un trou ménagé dans l'une des demi-coquilles de la boîte pour serrer la palette, rend pratiquement solidaire cette demi-coquille de la rampe de robinets du site de connexion et gêne les mouvements d'ouverture de l'autre demi-coquille. Il en résulte qu'avec la structure actuelle, les manipulations des robinets de la rampe sont très malaisées et parfois même impossibles. De plus, elles peuvent causer des blessures au personnel chargé de l'actionnement des robinets, ce qui va à l'encontre de l'asepsie recherchée.

La présente invention a pour but de remédier à cela en fournissant une structure de protection qui permette l'accès aisé à l'un quelconque des robinets de la rampe de robinets, sans que cela affecte l'asepsie.

A cet effet, dans la structure selon l'invention, les demi-coquilles sont articulées, par des charnières indépendantes, espacées verticalement et formées sur les bords longitudinaux amincis d'une paroi transversale postérieure, solidaire d'un coulisseau, ce coulisseau étant monté mobile en translation sur la palette liée au site de connexion dans la direction perpendiculaire au plan de la paroi transversale postérieure, tandis que la longueur de cette palette est au moins égale à la course nécessaire au coulisseau précité pour obtenir le dégagement total du site de protection par les deux demi-coquilles en position ouverte.

Ainsi, lorsque le personnel veut accéder à l'un quelconque des robinets, dès qu'il a actionné les moyens de fermeture élastique des deux demi-coquilles dans le sens du déverouillage, il peut en même temps faire coulisser ces deux demi-coquilles en direction de la pince de fixation pour dégager totalement le site de connexion. Il en résulte que l'accès au site de protection est facilité et que tout risque de blessure est supprimé. Après réglage des robinets, il suffit de rapprocher les deux demi-coquilles de la rampe et de les fermer sur cette rampe pour retrouver les conditions stériles recherchées.

Dans une forme de réalisation préférentielle de l'invention, les deux demi-coquilles du boîtier sont reliées à des moyens à ressorts aptes, lors de l'ouverture du boîtier, à déplacer les deux demi-coquilles sur la palette en direction de l'extrémité postérieure de cette palette.

De la sorte, dès que l'opérateur a actionné les moyens de fermeture dans le sens du déverouillage, les moyens à ressort déplacent d'eux-mêmes les demi-coquilles pour dégager la rampe de robinets.

D'autres caractéristiques et avantages ressortiront de la description qui suit en référence au dessin schématique annexé représentant à titre d'exemple non limitatif deux formes d'exécution de cette structure.
Figure 1 est une en perspective avec coupe partielle de la demi-coquille supérieure d'une première forme d'exécution de cette structure,
Figures 2 et 3 sont des vues en coupe transversale d'une autre forme d'exécution de ce boîtier respectivement lorsqu'il est en position de fermeture et lorsqu'il est en position d'ouverture.

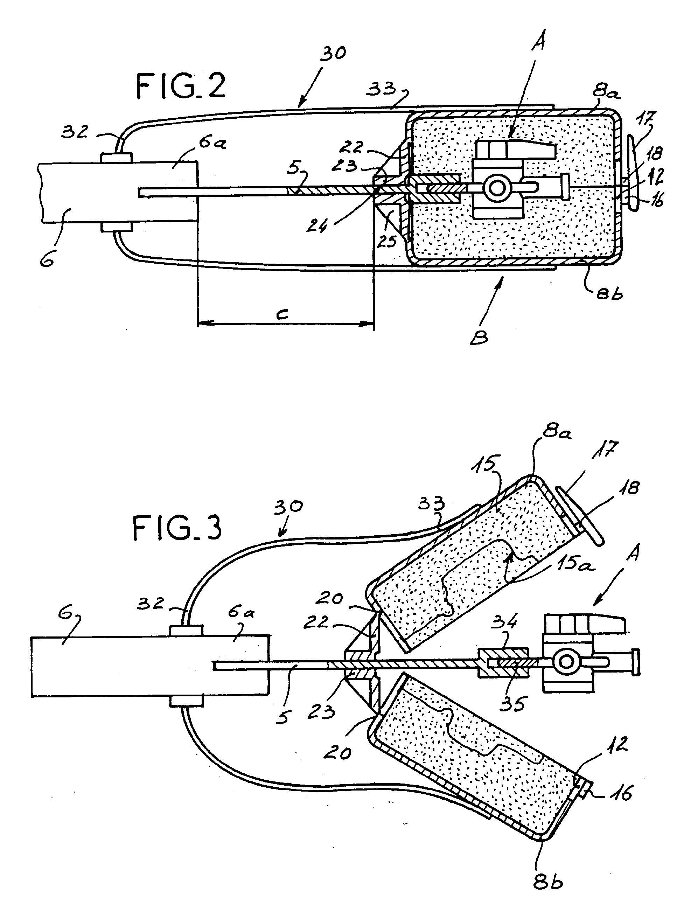

Aux figures, A désigne un site de connexion composé d'une tubulure principale 2, traversant en ligne droite le corps, et de plusieurs robinets 3 équipés chacun d'une tubulure transversale 4. La tubulure 2 est solidaire de l'une des extrémités d'une palette, 5 dont l'autre extrémité est solidaire d'une pince 6, de fixation au montant 7 d'une potence, ou d'un lit. Ce site de protection est protégé, de façon connue, par un boîtier qui désigné, de façon générale par B, est composé de deux demi-coquilles, respectivement, 8a et 8b. Chacune de ces demi-coquilles comporte, dans sa paroi frontale 9 et dans ses parois extrêmes 10, des ouvertures 12 pour le passage des tuyaux souples respectivement 13 et 14 se raccordant aux tubulures 4 et 2. Enfin, chaque demi-coquille est garnie par une mousse 15 en matière synthétique qui, comportant des empreintes 15a pour l'encastrement des éléments du site, est destinée à être imbibée par un agent antiseptique. La fermeture des deux éléments de boîtier 8a et 8b est verrouillée par un système de fermeture disposé sur les parois antérieures et montré aux figures 2 et 3. Ce système est composé de doigts 16 et de leviers 17 à articulation élastique 18.

Selon l'invention, chacune des deux demi-coquilles 8a et 8b n'est pas articulée directement à l'autre et par une unique charnière, comme cela est d'usage, mais sur les deux bords longitudinaux amincis 20, formant charnières, d'une paroi complémentaire 22, transversale et postérieure, solidaire d'un coulisseau 23. Ce coulisseau est traversé par une lumière 24 qui lui permet de se déplacer librement sur la palette 5, solidaire du site de connexion A, dans une direction perpendiculaire au plan de la paroi 22.

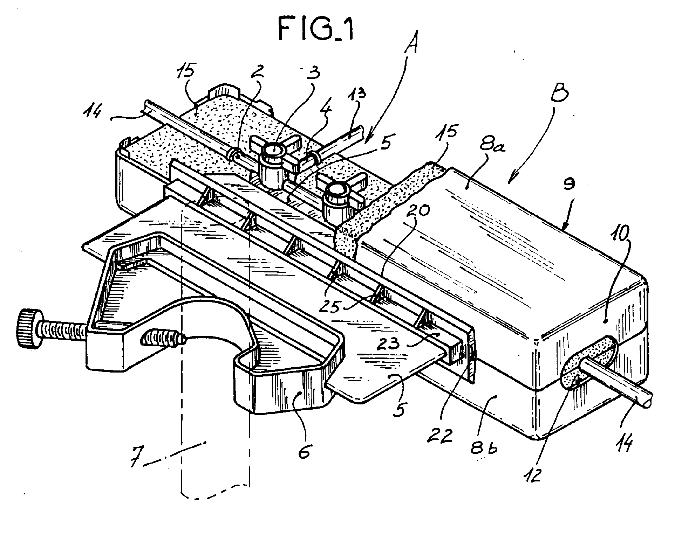

La figure 1 montre que, pour donner de la rigidité aux charnières ainsi constituées, et garantir une bonne fermeture des deux demi-boîtiers, la liaison entre la paroi 22 et le coulisseau 23 est renforcée par des goussets 25.

Grâce à cet agencement, et comme le montre la figure 3, lorsque l'opérateur actionne les moyens de verrouillage 16-17 des deux demi-coquilles 8a-8b pour procéder à l'ouverture en vue d'accéder au site de connexion A, il est très aisé, en maintenant sa pression sur l'une ou l'autre des deux demi-coquilles, de faire coulisser l'ensemble de ces deux demi-coquilles sur la palette 5 qui, à cet effet, a une longueur au moins égale à la course C qu'il faut faire effectuer au coulisseau, pour obtenir le dégagement total par les demi-coquilles du site de connexion A.

La forme d'exécution représentée aux figures 2 et 3 se différencie de la précédente par le fait que les deux demi-coquilles sont associées à des moyens à ressorts provoquant leur recul sur la palette dès que les moyens de verrouillage sont actionnés dans le sens du déverrouillage. Dans la forme d'exécution représentée, les moyens à ressort sont constitués par au moins une lame élastique en "U" 30, dont l'âme 32 est rendue solidaire du corps de fixation 6 ou de la palette 5, et dont les extrémités libres des branches 33 sont rendues solidaires de l'un ou l'autre des demi-boîtiers. Grâce à cela, dès que les systèmes de verrouillage sont actionnés dans le sens du déverrouillage, les branches 33 de la lame élastique tendent naturellement à s'écarter, ce qui a pour effet de rapprocher les extrémités libres de ces lames en direction du corps de fixation 6 et, en même temps, de provoquer le déplacement dans le même sens du coulisseau 23 et des deux demi-coquilles 8a-8b, comme le montre la figure 3. En d'autres termes, le déverrouillage des moyens de fermeture provoque le retrait automatique des deux demi-coquilles constituant le boîtier de protection du site de connexion A.

Dans les formes d'exécution représentées ci dessus, la palette 5 n'est pas monolithique avec le site de connexion, mais est rendue solidaire de celui-ci par enfourchement de la chape 34 ménagée à son extrémité antérieure sur la palette 35 solidaire de ce corps. De même, son bord postérieur est fixé au corps de fixation 6 par engagement dans la chape 6a de ce corps.

L'ensemble constitué par le boîtier, la rampe de connexion, la palette et le corps de fixation est réalisé par moulage de matière synthétique et est donc peu onéreux, ce qui permet de le jeter après une première utilisation.

Il ressort de ce qui précède que la structure de protection, selon l'invention, permet d'obtenir un excellent dégagement du site de connexion et facilite donc la tâche du personnel, en supprimant tout risque de blessure.

## Revendications

1. Structure de protection pour site de connexion à usage médical, comprenant un site de connexion (A) avec au moins un robinet (3) et des tubulures, ledite site de connexion étant solidaire d'une palette de fixation (5), un boîtier (B) composé de deux demi-coquilles (8a-8b) en matière synthétique, articulées par leurs parois longitudinales postérieures, munies sur leurs parois longitudinales antérieures de moyens de fermeture élastique (16-17), et munies de garnissages (15) en mousse, comportant des empreintes pour l'encastrement d'au moins un robinet et des tubulures du site de connexion, **caractérisée en ce que** les demi-coquilles (8a-8b) sont articulées, par des charnières (20), indépendantes, espacées verticalement et formées sur les bords longitudinaux amincis d'une paroi transversale postérieure (22), solidaire d'un coulisseau (23), ce coulisseau étant monté mobile en translation sur la palette (5) dans la direction perpendiculaire au plan de la paroi (22), tandis que la longueur de cette palette (5) est au moins égale à la course (C) nécessaire au coulisseau (23) pour obtenir le dégagement total du site de protection par les deux demi-coquilles (8a-8b) en position ouverte.

2. Structure selon la revendication 1, **caractérisée en ce que** les deux demi-coquilles (8a-8b) du boîtier (B) sont reliées à des moyens à ressort (30), aptes, lors de l'ouverture du boîtier (B), à déplacer les deux demi-coquilles de celui-ci sur la palette (5) en direction de l'extrémité postérieure de cette palette.

3. Structure selon la revendication 2, **caractérisée en ce que** les moyens à ressort sont constitués par au moins une lame élastique (30), cintrée en "U", dont l'âme (32) est solidaire du corps et dont les branches, disposées de part et d'autre de la palette (5), sont fixées par leur extrémité libre aux deux demi-coquilles (8a-8b).

## Patentansprüche

1. Schutzvorrichtung für eine Anschlußstelle zum medizinischen Gebrauch, umfassend eine Anschlußstelle (A) mit wenigstens einem Hahn (3) und Rohrabschnitten, wobei die Anschlußstelle mit einer Befestigungsplatte (5) verbunden ist, sowie ferner ein Gehäuse (B), welches aus zwei Kunststoffhalbschalen (8a, 8b) gebildet ist, die mittels ihrer hinteren Längswandungen angelenkt sind, die ferner an ihren vorderen Längswandungen mit elastischen Schließmittel (16-17) versehen sind, und die mit Schaumstoff-Auskleidungen (15) ausgestattet sind, wobei die Auskleidungen Ausnehmungen aufweisen zur Aufnahme wenigstens eines Hahns und von Rohrabschnitten der Anschlußstelle, **dadurch gekennzeichnet,** daß die Halbschalen (8a-8b) mittels unabhängiger Scharniere (20) angelenkt sind, welche vertikal beabstandet und an den dickenreduzierten Längsrändern einer mit einem Schlitten (23) verbundenen hinteren Querwandung (22) angeformt sind, wobei dieser Schlitten auf der Platte (5) in einer zur Ebene der Wandung (22) orthogonalen Richtung verschiebbar angeordnet ist, während die Länge dieser Platte (5) wenigstens gleich dem Weg (C) ist, den der Schlitten (23) benötigt, um in der Öffnungsstellung die totale Freigabe der Schutzstelle durch die beiden Halbschalen (8a-8b) zu erreichen.

2. Vorrichtung nach Anspruch 1, **dadurch gekennzeichnet,** daß die beiden Halbschalen (8a-8b) des Gehäuses (B) mit Federmitteln (30) verbunden sind, welche beim Öffnen des Gehäuses (B) die beiden Halbschalen desselben auf der Platte (5) in Richtung zum hinteren Ende dieser Platte hin verschieben können.

3. Vorrichtung nach Anspruch 2, **dadurch gekennzeichnet,** daß die Federmittel von wenigstens einem U-förmig gebogenen elastischen Blatt (30) gebildet sind, dessen Basis (32) mit dem Körper verbunden ist und dessen beidseits der Platte (5) angeordnete Arme mit ihren freien Enden an den beiden Halbschalen (8a-8b) befestigt sind.

## Claims

1. A protective structure for a connection site for medical use, including a connection site (A) with at least one tap (3) and connectors, said connecting site being connected to a fixing plate (5), a housing (B) comprising two half-shells (8a-8b) of synthetic material, articulated by their rear longitudinal walls, provided on their front longitudinal walls with resilient closure means (16-17), and provided with packing (15) of foam having impressions for embedding at least one tap and connectors of the connecting site, characterised in that the half-shells (8a, 8b) are articulated by hinges (20) which are independent, vertically spaced and formed on the thinned longitudinal edges of a rear transverse wall (22), connected to a slide (23), this slide being mounted to be movable in translation on the plate (5) in the direction perpendicular to the plane of the wall (22), whilst the length of this plate (5) is at least equal to the travel (C) necessary for the slide (23) to obtain the complete disengagement from the protective site by the two half-shells (8a-8b) in the open position.

2. A structure according to Claim 1, characterised in that the two half-shells (8a-8b) of the housing (B) are connected to spring means (30) adapted, when the housing (B) is opened, to move the two half-shells thereof on the plate (5) in the direction of the rear end of this plate.

3. A structure according to Claim 2, characterised in that the spring means are constituted by at least one resilient blade (30) bent into a "U", of which the core (32) is connected to the body and of which the arms, disposed to either side of the plate (5), are fixed by their free ends to the two half-shells (8a-8b).
